# EUROPÄISCHE PATENTSCHRIFT

(11) **EP 3 419 719 B1**
(45) Veröffentlichungstag und Bekanntmachung des Hinweises auf die Patenterteilung: **06.11.2024**
(21) Anmeldenummer: 17706717.0
(22) Anmeldetag: 20.02.2017
(51) Int. Cl.: A61F 2/14, A61N 1/36, A61N 1/378

(54) **VORRICHTUNG UND VERFAHREN ZUR VERSORGUNG EINES AKTIVEN AUGENIMPLANTATS**
DEVICE AND METHOD FOR SUPPLYING ENERGY TO AN ACTIVE OCULAR IMPLANT
DISPOSITIF ET PROCÉDÉ D'ALIMENTATION D'UN IMPLANT OCULAIRE ACTIF

(30) Priorität: 24.02.2016 DE 102016103285
(43) Veröffentlichungstag der Anmeldung: 02.01.2019
(73) Patentinhaber: Carl Zeiss AG, 73447 Oberkochen (DE)
(72) Erfinder: KINDT, Johannes, 99425 Weimar (DE); DOBSCHAL, Hans-Jürgen, 99510 Kleinromstedt (DE)
(74) Vertreter: Kraus & Lederer PartGmbB
(86) Internationale Anmeldenummer: PCT/EP2017/053812
(87) Internationale Veröffentlichungsnummer: WO 2017/144421

(56) Entgegenhaltungen:
- DE-A1- 10 315 397
- US-A1- 2015 182 748

## Beschreibung

Die vorliegende Anmeldung betrifft Vorrichtungen und Verfahren zum Versorgen eines aktiven Augenimplantats mit Energie mittels Infrarotstrahlung.

Aktive Augenimplantate sind Vorrichtungen, welche in ein Auge eines Patienten implantiert werden, um dort bestimmte Funktionen auszuüben. Ein Beispiel für derartige aktive Augenimplantate sind Retinaimplantate. Retinaimplantate werden entwickelt, um Personen, denen die Sehfähigkeit abhanden gekommen ist, bei welchen jedoch noch eine Verbindung von dem Sehnerv zum Gehirn besteht, das Sehvermögen zumindest bis zu einem gewissen Grad wiederzugeben. Derartige Retinaimplantate umfassen üblicherweise einen Bildsensor, durch welchen - gegebenenfalls mit zusätzlichen Schaltungen - elektrische Impulse erzeugt werden, die dann über den Sehnerv registriert werden.

Andere Beispiele für aktive Augenimplantate sind aktiv akkommodierende Intraokularlinsen oder implantierte Sensoren zur Messung von Parametern im Auge, beispielsweise des Blutzuckerspiegels im Kammerwasser. Derartige aktive Augenimplantate benötigen im Gegensatz zu passiven Implantaten (z.B. einfachen Linsen) elektrische Energie, um betrieben zu werden.

Eine Möglichkeit der Versorgung mit Energie ist die Zuführung von Infrarotstrahlung unterhalb des sichtbaren Bereichs, welche dann durch das aktive Augenimplantat im Wesentlichen mittels einer Solarzelle oder ähnlichen Einrichtung in elektrische Energie umgewandelt wird.

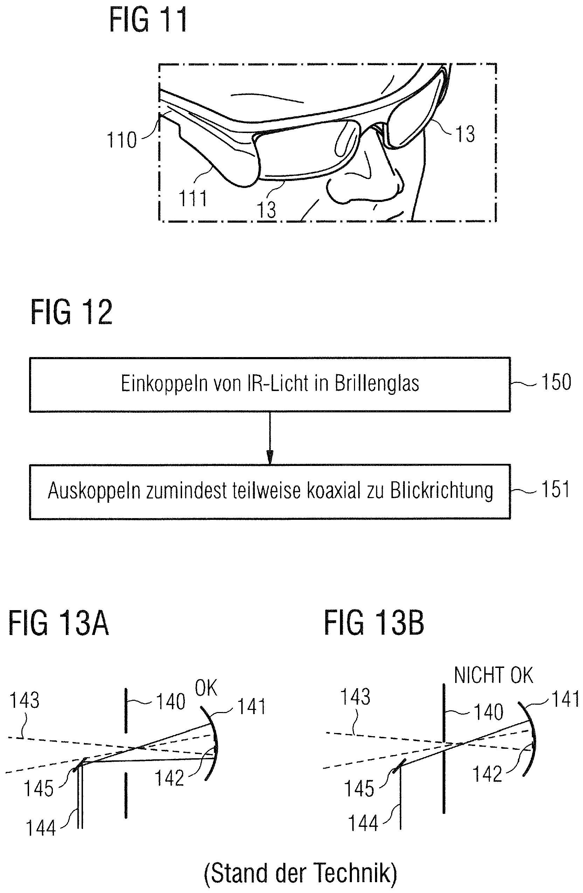

Bei einem herkömmlichen Ansatz geschieht dies dadurch, dass Infrarotstrahlung von einer Infrarotlichtquelle, beispielsweise einem Infrarotlaser, über ein Umlenkelement wie ein Prisma oder einen Spiegel leicht schräg in das Auge eingekoppelt wird. Dies ist in den Fig. 13A und 13B dargestellt. In den Fig. 13A und 13B wird ein Infrarotstrahl 144 über ein Umlenkelement 145, beispielsweise ein Prisma, durch eine Irisblende 140 eines Auges 141 gelenkt. Ein derartiges Umlenkelement 145 kann beispielsweise an einem Rand eines Brillenglases einer entsprechenden Brille angeordnet sein. Mit 143 sind Strahlen (z.B. sichtbares Licht) bezeichnet, die einer Hauptblickrichtung des Auges, beispielsweise einer optischen Achse bei einem Geradeausschauen, entsprechen.

Ist die Irisblende 140 weit genug geöffnet, wie in Fig. 13A gezeigt, trifft der Infrarotstrahl 144 in den Bereich eines Retinaimplantates 142, welches hier als Beispiel für ein aktives Augenimplantat dient und welches sich in dem Auge 141 befindet, und kann dieses somit mit Energie versorgen. Wenn jedoch wie in Fig. 13B gezeigt die Irisblende 140 verengt, d.h. geschlossen ist, gelangt der Infrarotstrahl neben das Implantat 142 in das Auge, so dass das Implantat nicht mehr mit Energie versorgt werden kann. Weiterhin kann eine Veränderung der Blickrichtung zu einer Abschattung des Infrarotstrahls durch die Iris führen, besonders, wenn diese verengt ist.

Somit ist es bei dieser herkömmlichen Herangehensweise gegebenenfalls nötig, die Irisblende operativ zu vergrößern, was einen zusätzlichen operativen Eingriff beispielsweise beim Einsetzen des Implantats 142 oder sogar eine zusätzliche Operation benötigt. Weiterhin wirkt sich die permanente Vergrößerung der Iris negativ auf die Fähigkeit des Auges zur Anpassung an verschiedene Lichtverhältnisse sowie das ästhetische Operationsergebnis aus.

Aus der DE 103 15 397 A1 sowie der US 2015/182748 A1 sind Vorrichtungen gemäß dem Oberbegriff des Anspruchs 1 bzw. Verfahren gemäß dem Oberbegriff des Anspruchs 11 bekannt. Dabei erfolgt jeweils eine Defokussierung von Infrarotstrahlenbündeln, die zum Auge hin gelenkt werden.

Es ist daher eine Aufgabe der vorliegenden Erfindung, Vorrichtungen und Verfahren zur Energieversorgung eines aktiven Augenimplantats bereitzustellen, welche auch bei vergleichsweise kleinen Irisblenden und/oder bei variierender Blickrichtung funktionsfähig sind.

Erfindungsgemäß werden eine Vorrichtung nach Anspruch 1 sowie ein Verfahren nach Anspruch 9 bereitgestellt. Die Unteransprüche definieren weitere Ausführungsformen.

Gemäß einem ersten Aspekt wird eine Vorrichtung zur Versorgung eines aktiven Augenimplantats, z.B. eines Retinaimplantats, in einem Auge mit Energie bereitgestellt, umfassend:
eine Infrarotlichtquelle,
ein Brillenglas, wobei die Infrarotlichtquelle derart angeordnet ist, dass von der Infrarotlichtquelle ausgehendes Licht zu einer in oder auf dem Brillenglas angeordneten Lenkeinrichtung gelangt, welche das Infrarotlicht zu einem Auge eines Benutzers hin lenkt.

Erfindungsgemäß ist die Lenkeinrichtung eingerichtet, das Infrarotlicht als ein oder mehrere fokussierte Strahlenbündel zu dem Auge hin zu lenken, z.B. auszukoppeln.

Durch die Anordnung der Lenkeinrichtung kann die Richtung, aus der das Infrarotlicht auf das Auge trifft, bestimmt werden.

Hierzu kann insbesondere das Infrarotlicht in das Brillenglas eingekoppelt werden, welches dann als Wellenleiter fungiert, um das Infrarotlicht zu der Lenkeinrichtung zu lenken. Diese fungiert dann als Auskoppeleinrichtung und koppelt das Licht zu dem Auge hin aus.

Durch das Einkoppeln der Infrarotstrahlung in das Brillenglas und die Bereitstellung des Auskoppelelements kann die Richtung, aus der das Infrarotlicht auf das Auge trifft, gegenüber der schrägen Einkopplung des Standes der Technik flexibel gewählt werden. Gegenüber einer Freistrahloptik ergibt sich außerdem der Vorteil, dass durch die Lichtleitung in dem Brillenglas Abschattungen durch Wangenknochen, Wimpern, Augenbrauen, Haare und dergleichen vermieden werden können.

Die Lenkeinrichtung kann für sichtbares Licht transparent sein, um so eine ungehinderte Sicht zu ermöglichen.

Die Infrarotlichtquelle emittiert bevorzugt im Wesentlichen (d.h. z.B. bis auf unerwünschte Effekte, z.B. unvollständige Filterung) nur im infraroten Bereich oberhalb von 780 nm, um die Wahrnehmung von sichtbarem Licht nicht zu stören.

Die Lenkeinrichtung kann eingerichtet sein, das Infrarotlicht zumindest teilweise koaxial zu einer Blickrichtung des Auges zum Auge hin zu lenken, z.B. aus dem Brillenglas auszukoppeln.

Durch die koaxiale Einkopplung kann auch bei kleiner Iris die Versorgung des aktiven Augenimplantats sichergestellt werden.

Die Lenkeinrichtung kann eingerichtet sein, das Infrarotlicht als eine Vielzahl von Strahlenbündeln mit unterschiedlichen Richtungen zu dem Auge hin zu lenken, z.B. aus dem Brillenglas auszukoppeln.

Durch die Verwendung mehrerer Richtungen kann eine Versorgung des aktiven Augenimplantats auch bei Augenbewegung sichergestellt werden. Dies ist auch ein Vorteil gegenüber einer Freistrahloptik mit einer Umlenkung oder dergleichen an einer Stelle, bei der nur eine Richtung erzeugt wird.

Ein oder mehrere Fokuspunkte der ein oder mehreren Strahlenbündel liegen nahe bei einer Pupille (durch eine Irisblende definiert) des Auges.

Bei einer anderen, nicht beanspruchten Implementierung können ein oder mehrere Fokuspunkte der ein oder mehreren Strahlenbündel können nahe eines Drehpunktes/Augenmittelpunktes des Auges liegen. Der Drehpunkt ist dabei ein Punkt, um den sich das Auge dreht.

"Nahe bei" bedeutet im Rahmen dieser Anmeldung dabei einen Bereich von ±5 mm, insbesondere ±4 mm ±3 mm, ±2 mm oder ±1 mm zu dem jeweiligen Punkt oder Ort (Pupille oder Drehpunkt/Augenmittelpunkt).

Die Lenkeinrichtung kann ein Transmissionsgitter, ein Reflexionsgitter, einen teildurchlässigen Strahlteiler, einen wellenlängenselektiven Strahlteiler, ein Volumenhologramm und/oder ein Fresnel-Element umfassen.

Durch ein Fresnel-Element oder ein optisches Gitter oder auch durch einen wellenlängenselektiven Strahlteiler kann die Auskopplung auf vergleichsweise einfache Weise erfolgen, ohne dass die Sicht durch das Brillenglas wesentlich behindert wird.

Das Transmissionsgitter und/oder das Reflexionsgitter können ein holografisches Gitter umfassen.

Gemäß einem zweiten Aspekt wird ein Verfahren zur Versorgung eines aktiven Augenimplantats in einem Auge mit Energie bereitgestellt, umfassend:
Lenken von Infrarotlicht zu einer Lenkeinrichtung in oder auf einem Brillenglas, und
Lenken des Infrarotlichts von der Lenkeinrichtung zu einem Auge eines Benutzers hin. Erfindungsgemäß wird das Infrarotlicht von der Lenkeinrichtung als ein oder mehrere fokussierte Strahlenbündel zu dem Auge hin gelenkt.

Das Verfahren kann insbesondere durch Einkoppeln von Infrarotlicht in ein Brillenglas, und Auskoppeln des Infrarotlichts zu einem Auge eines Benutzers hin realisiert werden.

Das Lenken bzw. Auskoppeln kann ein Lenken bzw. Auskoppeln an verschiedenen Stellen des Brillenglases unter verschiedenen Winkeln zu dem Auge hin umfassen.

Die ein oder mehreren fokussierten Strahlenbündel können einen Fokuspunkt nahe bei einer Pupille des Auges aufweisen.

Das Lenken bzw. Auskoppeln kann bei einer nicht beanspruchten Implementierung ein Lenken bzw. Auskoppeln als ein oder mehrere Strahlenbündel mit einem Fokuspunkt jeweils in der Nähe des Drehpunktes des Auges umfassen.

Das Verfahren kann mit einer der oben beschriebenen Vorrichtungen durchgeführt werden.

Die Erfindung wird nachfolgend unter Bezugnahme auf die beigefügte Zeichnung anhand von Ausführungsbeispielen detailliert erläutert. Es zeigen:
Fig. 1 eine schematische Querschnittsansicht einer Vorrichtung gemäß einem Ausführungsbeispiel,
Fig. 2A und 2B Darstellung zur Veranschaulichung von Vorteilen des Ausführungsbeispiels der Fig. 1,
Fig. 3 eine Darstellung zur Veranschaulichung einer Augenbewegung,
Fig. 4 eine schematische Darstellung zur Veranschaulichung einer Einkopplung aus verschiedenen Richtungen,
Fig. 5A eine Draufsicht auf ein Brillenglas gemäß einem Ausführungsbeispiel,
Fig. 5B eine Querschnittsansicht der Fig. 5A entlang einer Linie A-A,
Fig. 6 eine Querschnittsansicht einer Vorrichtung gemäß einem Ausführungsbeispiel,
Fig. 7 eine Querschnittsansicht einer Vorrichtung gemäß einem weiteren Ausführungsbeispiel,
Fig. 8-10 verschiedene Ansichten einer Vorrichtung gemäß einem Ausführungsbeispiel mit verschiedenen Augenstellungen,
Fig. 11 eine Ansicht einer Brille gemäß einem Ausführungsbeispiel,
Fig. 12 ein Flussdiagramm zur Veranschaulichung eines Verfahrens gemäß einem Ausführungsbeispiel,
Fig. 13A und 13B Diagramme zur Erläuterung einer herkömmlichen Herangehensweise,
Fig. 14 eine Querschnittsansicht einer Vorrichtung gemäß einem weiteren Ausführungsbeispiel.

Im Folgenden werden verschiedene Ausführungsbeispiele detailliert erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente oder Komponenten, weniger Elemente oder Komponenten oder auch zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein.

Zur Vermeidung von Wiederholungen werden gleiche oder einander entsprechende Elemente in verschiedenen Figuren mit dem gleichen Bezugszeichen bezeichnet und werden nicht mehrmals erläutert.

Bei den folgenden Ausführungsbeispielen wird ein Retinaimplantat als Beispiel für ein aktives Augenimplantat verwendet. Die beschriebenen Techniken sind jedoch auch auf andere aktive Augenimplantate, beispielsweise die eingangs erwähnten Augenimplantate, anwendbar.

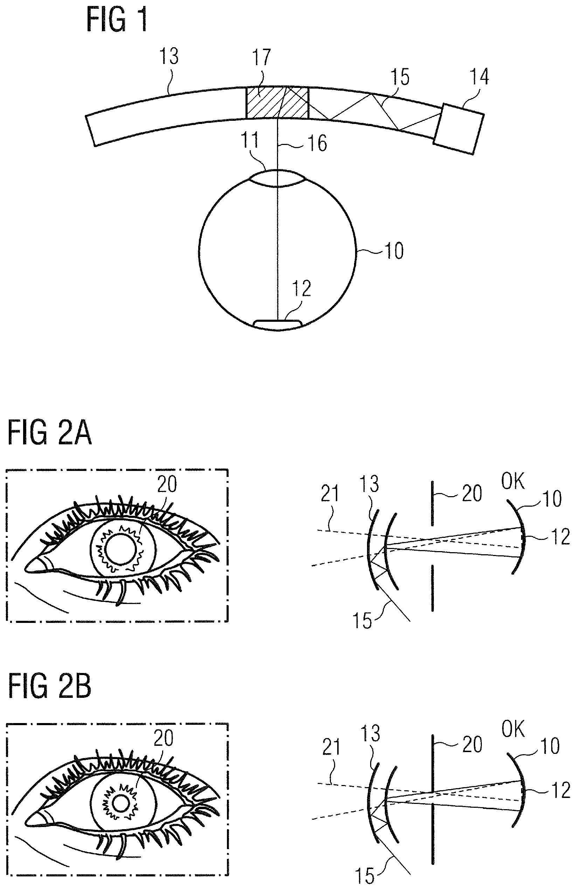

In Fig. 1 ist eine Vorrichtung gemäß einem Ausführungsbeispiel schematisch dargestellt. Die Vorrichtung des Ausführungsbeispiels der Fig. 1 dient dabei dazu, ein Retinaimplantat 12 in einem Auge 10 eines Benutzers mittels Infrarotstrahlung mit Energie zu versorgen. Diese Infrarotstrahlung kann dann von dem Retinaimplantat 12 mittels einer Solarzelle oder dergleichen in elektrische Energie umgewandelt werden.

Die Vorrichtung der Fig. 1 umfasst ein Brillenglas 13, welches vor dem Auge 10 anzuordnen ist. Unter einem Brillenglas wird dabei im Rahmen der vorliegenden Anmeldung allgemein ein transparentes Medium verstanden, welches mittels einer entsprechenden Halterung vor dem Auge 10 angeordnet werden kann. Ein derartiges Brillenglas 13 kann aus einem Glas oder auch einem Kunststoffmaterial bestehen. Das Brillenglas kann entlang einer oder zwei Richtungen gekrümmt ausgeführt oder plan sein. Das Brillenglas kann eine optische Brechkraft aufweisen. Des Weiteren umfasst die Vorrichtung der Fig. 1 eine Infrarotlichtquelle 14. Als Infrarotlichtquelle 14 kann beispielsweise eine Infrarotleuchtdiode (LED; oder mehrere LEDs) verwendet werden, aber es können auch andere geeignete Lichtquellen, gegebenenfalls in Verbindung mit Filtern, um sichtbares Licht herauszufiltern, verwendet werden. Bevorzugt emittiert die Infrarotlichtquelle 14 (gegebenenfalls durch den Einsatz entsprechender Filter) im Wesentlichen nur infrarotes Licht und im Wesentlichen kein Licht im sichtbaren Bereich, um eine mittels des Retinaimplantats 12 vorgenommene Wahrnehmung der Umgebung nicht zu stören. Die Infrarotlichtquelle 14 kann insbesondere einen Infrarotlaser umfassen. Ein InfrarotLaser, beispielsweise eine Infrarot Halbleiter-Laserdiode besitzt eine besonders schmale Bandbreite hinsichtlich des emittierten Lichts, was für die Funktion der Lenkeinrichtung hilfreich sein kann. Bei Einsatz eines Filters kann "im Wesentlichen" "im Rahmen der Güte des Filters" bedeuten. Die Wellenlänge des Infrarotlichts kann dabei insbesondere über 780 nm liegen. Das Infrarotlicht 15, welches von der Infrarotlichtquelle 14 emittiert wird, kann monochromatisch sein, d.h. im Wesentlichen nur eine Wellenlänge aufweisen, oder auch eine breitere Spektralverteilung, beispielsweise in der Größenordnung von etwa 30 nm, aufweisen.

Das Infrarotlicht 15 wird dabei in das Brillenglas 13 derart eingekoppelt, dass das Brillenglas 13 als Wellenleiter für das Infrarotlicht dient. Eine derartige Wellenleitung erfolgt im Wesentlichen durch Totalreflexion an inneren Oberflächen des Brillenglases 13, wofür der Einkopplungswinkel des Infrarotlichts 15 entsprechend zu wählen ist, um die Bedingung für Totalreflexion zu erfüllen. Wird die Bedingung nicht erfüllt, kommt es zu höheren Verlusten von Infrarotlicht. Zwischen der Infrarotlichtquelle 14 und dem Brillenglas 13 kann optional eine Strahlformungsoptik angeordnet sein, um das von der Lichtquelle emittierte Licht vor der Einkopplung in das Brillenglas geeignet zu formen, beispielsweise zu kollimieren. Die Strahlformungsoptik kann außerdem weitere optische Elemente wie Wellenplättchen oder Streuscheiben enthalten.

Zudem umfasst die Vorrichtung der Fig. 1 eine Auskoppeleinrichtung 17 (ein Beispiel für eine Lenkeinrichtung), welche in dem Brillenglas 13 angeordnet ist und das Infrarotlicht 15 als ausgekoppeltes Licht 16 in Richtung einer Pupille 11 des Auges hin auskoppelt. Die Auskoppeleinrichtung 17 ist dabei wellenlängenselektiv, so dass das Infrarotlicht ausgekoppelt wird, jedoch der entsprechende Bereich des Brillenglases 13, in dem die Auskoppeleinrichtung 17 angeordnet ist, im sichtbaren Spektrum transparent bleibt, so dass das Retinaimplantat 12 den Bildeindruck der zu betrachtenden Umgebung ohne Störung aufnehmen kann.

Die Auskopplung erfolgt dabei bevorzugt so, dass zumindest ein Teil des ausgekoppelten Lichtes 16 koaxial zu einer Blickrichtung des Auges 10, d.h. zu der optischen Achse des Auges 10 in Blickrichtung, liegt. Dies kann insbesondere für eine Hauptblickrichtung (z.B. Geradeaussehen) der Fall sein. Bevorzugt erfolgt die Auskopplung, wie im Folgenden noch näher erläutert werden wird, so, dass für einen möglichst großen Blickwinkelbereich des Auges 10 das Implantat beleuchtet wird und somit aktiv bleibt. Die Auskoppeleinrichtung 17 kann dabei auf verschiedene Art und Weise realisiert werden. Beispielsweise kann ein optisches Gitter, insbesondere ein optisches Gitter in Reflexion auf einer von dem Auge 10 abgewandten Oberfläche des Brillenglases 13, ein optisches Gitter in Transmission auf einer dem Auge 10 zugewandten Seite des Brillenglases 13, eine wellenlängenselektiv verspiegelte Fresnel-Struktur oder ein herkömmlicher wellenlängenspezifischer, z.B. dichroitischer, Strahlteiler verwendet werden. Auch Kombinationen hiervon sind möglich. Die Auskoppeleinrichtung in Form einer Fresnel-Struktur, eines optischen Gitters in Transmission oder eines optischen Gitters in Reflexion kann bei manchen Ausführungsbeispielen im Inneren des Brillenglases angeordnet sein (beispielsweise zum Schutz dieser Auskoppeleinrichtung vor Beschädigungen oder Verschmutzung).

Durch eine Auskopplung wie in Fig. 1 koaxial zu einer Blickrichtung des Auges kann im Gegensatz zu der unter Bezugnahme auf Fig. 13 in der Einleitung erläuterten herkömmlichen Herangehensweise auch bei einer vergleichsweise kleinen Irisöffnung eine Beleuchtung und somit Energieversorgung des Retinaimplantats 12 sichergestellt werden. Dies wird unter Bezugnahme auf die Fig. 2A und 2B veranschaulicht, welche ähnlich den Fig. 13A und 13B sind.

In Fig. 2A ist die Situation für das Auge 10 mit einer relativ weit geöffneten Iris 20 dargestellt. In Fig. 2B ist eine Situation für eine weiter geschlossene Iris 20 dargestellt. In beiden Fällen gelangt sowohl das Infrarotlicht 15 als auch sichtbares Licht 21 aus der Umgebung zu dem Retinaimplantat 12 in dem Auge 10.

Somit kann mit dem in Fig. 1 dargestellten Ausführungsbeispiel beispielsweise eine operative Öffnung der Irisblende, um das Retinaimplantat 12 ausreichend zu versorgen, vermieden werden.

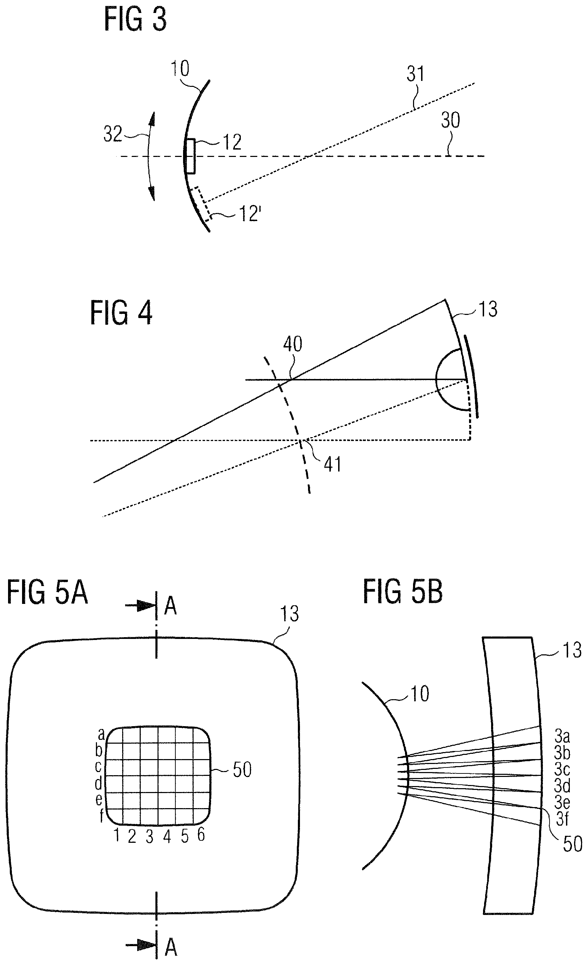

Wie bereits erläutert, erfolgt bevorzugt die Auskopplung des Infrarotlichts aus dem Brillenglas bevorzugt derart, dass das Implantat für einen möglichst großen Blickwinkelbereich beleuchtet wird, z.B. von verschiedenen Orten aus bzw. aus einem breiteren Bereich heraus. Dies wird nunmehr unter Bezugnahme auf die Fig. 3-5 näher erläutert.

Werden Objekte in verschiedenen Richtungen betrachtet, bewegt sich das Auge und es kommt zu einer Änderung der Blickrichtung und optischen Achse des Auges. Dies ist schematisch in Fig. 3 dargestellt. Bei einer ersten Blickrichtung entsprechend einer gestrichelten Linie 30 befindet sich das Implantat in einer Position 12. Bewegt sich das Auge in einer Drehbewegung entsprechend einem Pfeil 32, kann das Implantat beispielsweise wie als 12' dargestellt zu liegen kommen, entsprechend einer Blickrichtung 31. Durch eine entsprechende Auskopplung kann sichergestellt werden, dass in beiden Positionen das Retinaimplantat ausreichend mit Infrarotlicht beleuchtet wird, um es mit Energie zu versorgen.

Die Fig. 4 zeigt dabei ein Beispiel mit zwei verschiedenen Auskopplungsrichtungen aus einem Brillenglas 13. Insbesondere weist das Brillenglas in Fig. 4 als Beispiel zwei Zonen auf, aus denen Licht entsprechend zwei verschiedener Blickrichtungen ausgekoppelt wird. Dies führt zu zwei verschiedenen Foki (Fokuspunkten) 40, 41, welche bevorzugt nahe der Irisblende liegen, so dass ein Durchgang auch durch eine vergleichsweise weit geschlossene Irisblende (d.h. die durch die Irisblende definierte Pupille) ermöglicht wird.

Typischerweise ist ein Retinaimplantat beispielsweise 4-8 mm breit, und ein Pupillendurchmesser bei Helladaption, d.h. relativ weit geschlossener Iris, kann 2-3 mm betragen. Somit ist es bevorzugt, dass ein Fokuspunkt der Auskopplung wie die Punkte 40 und 41 in Fig. 4 nahe bei der Iris liegt, da durch ein kollimiertes Strahlenbündel (auch als Beleuchtungsstrahl bezeichnet) mit Durchmesser der Pupille keine vollständige Ausleuchtung des Implantats möglich wäre, und durch ein beispielsweise auf den Augenmittelpunkt (Drehpunkt des Auges) fokussiertes Strahlenbündel, wobei der Augenmittelpunkt zu Iris und Implantat den gleichen Abstand hat, eine zu große Abschattung durch die Iris erfolgen würde. Es wird also ein nahe vor oder hinter der Iris und somit der Pupille fokussierter (also im Bereich von ±5 mm, insbesondere ±4 mm, ±3 mm, ±2 mm oder ±1 mm zur Pupille fokussierter) divergenter Strahlengang oder eine Vielzahl derartiger Strahlengänge verwendet. Der Fokuspunkt oder die Fokuspunkte liegen dabei bevorzugt ca. 13-30 mm von einer dem Auge zuzuwendenden Oberfläche des Brillenglases entfernt, entsprechend typischen Abständen zwischen Brillenglas und Iris im Bereich von 16-27mm. Andere Werte sind ebenso möglich.

Bei anderen Implementierungen, die keine beanspruchten Ausführungsbeispiele sind, kann auch auf oder nahe dem Augenmittelpunkt fokussiert werden, z.B. in einem Bereich von ±5 mm, ±4 mm, ±3 mm, ±2 mm oder ±1 mm vor oder hinter dem Augenmittelpunkt. Dies kann zwar wie oben erwähnt zu Abschattungen führen, jedoch kann so eine höhere Toleranz gegenüber z.B. lateralen Verschiebungen des Auges zum Beleuchtungsstrahl erzielt werden. Welche Möglichkeit gewählt wird (Fokussierung nahe bei der Pupille/Iris oder nahe beim Augenmittelpunkt) kann in Abhängigkeit von den Gegebenheiten der jeweiligen Implementierung und des jeweiligen Augenimplantats, z.B. abhängig von einem Energiebedarf des Augenimplantats oder einzuhaltenden Grenzwerten für die Energie des Beleuchtungsstrahls.

Während in Fig. 4 nur zwei derartige Strahlengänge dargestellt werden, kann eine Vielzahl derartiger Strahlengänge verwendet werden. Ein Beispiel hierfür ist in Fig. 5 gezeigt. Dabei zeigt die Fig. 5A eine schematische Draufsicht auf ein Brillenglas 13, und die Fig. 5B zeigt eine Querschnittsansicht entlang einer Linie A-A der Fig. 5A.

Das Brillenglas 13 der Fig. 5A und 5B weist dabei eine Anordnung von 36 Auskopplungselementen auf, welche in einer 6x6 Matrix 50 angeordnet sind und somit die Auskopplungseinrichtung 17 der Fig. 1 bilden. Jedes der Auskopplungselemente der Matrix 50 erzeugt ein Strahlenbündel, welches wie oben erläutert bevorzugt einen Fokus in der Nähe der Pupille bzw. Iris aufweist. Von jedem der Auskopplungselemente wird Licht aus einem anderen Winkel zu dem Auge 10 hin gelenkt, so dass bei verschiedenen Blickrichtungen das Implantat 12 beleuchtet werden kann. Während in den Fig. 5A und 5B eine 6x6 Matrix von Auskoppelelementen dargestellt ist, ist dies nur als Beispiel zu verstehen, und je nach Platz auf dem Brillenglas und zur Verfügung stehendem IR-Licht kann jede beliebige Anordnung verwendet werden. Im Wesentlichen kann ein Übergang zu einer kontinuierlichen Auskopplung über einen gewissen Bereich stattfinden, bei dem aus jedem Punkt der Brillenglasoberfläche Infrarotlicht in einem bestimmten Winkelbereich ausgekoppelt wird. Dabei ist dieser Winkel bevorzugt so gewählt, dass möglichst wenig Licht ausgekoppelt wird, welches das Retinaimplantat in keiner Stellung des Auges 10 erreichen kann, um Verluste möglichst gering zu halten und eine insgesamt benötigte Lichtenergie zu begrenzen. Auf diese Weise kann eine Lebensdauer einer Stromversorgung (beispielsweise Batterie) für die Infrarotlichtquelle vergrößert werden, und die Betriebssicherheit der Brille kann erhöht werden, da geringere Leistungen notwendig sind.

Eine derartige Auskopplung in einen definierten Winkelbereich kann z.B. durch Variation einer Gitterkonstanten eines auskoppelnden Gitters, erreicht werden. Eine andere Möglichkeit ist eine polychromatische Beleuchtung eines Gitters mit konstanter Gitterkonstante, wobei sich der Winkel je nach Wellenlänge des Lichts ändert. Eine derartige polychromatische Beleuchtung kann beispielsweise eine spektrale Breite in der Größenordnung von 30 nm aufweisen, ist jedoch nicht hierauf beschränkt.

Eine andere Möglichkeit ist eine Krümmung von auskoppelnden Flächen einer als Auskoppeleinrichtung verwendeten Fresnel-Struktur die insbesondere wellenlängenselektiv verspiegelt und/oder, mit optischem Medium (z.B. des Brillenglases) aufgefüllt sein kann. Auch Kombinationen dieser Herangehensweisen sind möglich.

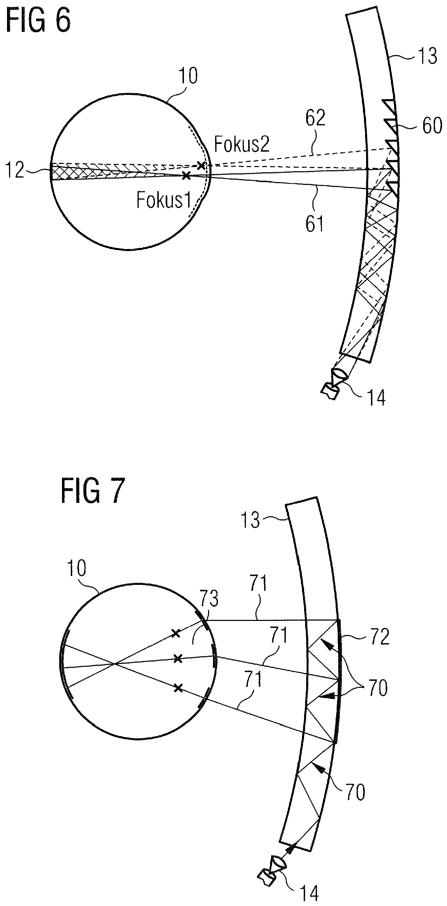

Als nächstes werden unter Bezugnahme auf die Fig. 6 und 7 zwei Ausführungsbeispiele für erfindungsgemäße Vorrichtungen noch näher erläutert.

Bei dem Ausführungsbeispiel der Fig. 6 umfasst die Infrarotlichtquelle 14 beispielsweise eine oder mehrere Infrarotleuchtdioden, welche ein polychromatisches Infrarotlichtbündel beispielsweise mit einer Halbwertsbreite von 30 nm (FWHM, vom Englischen "Full Width Half Maximum") erzeugt. Es können jedoch auch andere Lichtquellen verwendet werden. Als Auskoppeleinrichtung umfasst die Vorrichtung der Fig. 6 ein Fresnel-Element 60, wobei verschiedene Facetten des Fresnel-Elements Lichtstrahlen mit verschiedenem Fokus nahe bei einer Iris erzeugen können, um das Retinaimplantat 12 insbesondere bei verschiedenen Augenstellungen zu beleuchten. Die Facetten des Fresnel-Elements können wellenlängenselektiv verspiegelt sein. Das Fresnel-Element 60 kann mit dem Material des Brillenglases 13 aufgefüllt sein. Als Beispiel sind ein erster Lichtstrahl 31 mit einem ersten Fokus (Fokus 1) und ein zweites Strahlenbündel 62 mit einem zweiten Fokus (Fokus 2) eingezeichnet, wobei weitere Facetten weitere Strahlenbündel erzeugen können. Die Facetten des Fresnel-Elements 60 können dabei leicht gekrümmt sein, um Strahlenbündel mit gewünschtem Fokus zu erzeugen. Das von der Lichtquelle 14 erzeugte Licht ist dabei leicht divergent.

In Fig. 7 ist ein weiteres Ausführungsbeispiel dargestellt. Bei dem Ausführungsbeispiel der Fig. 7 ist an einer dem Auge abgewandten Oberfläche des Brillenglases 13 ein Gitter 70 (Reflexionsgitter), welches insbesondere als holografisches Gitter implementiert sein kann, angeordnet. Strahlenbündel werden dabei als Auskopplungen 71 erster Ordnung durch das Reflexionshologramm 72 erzeugt, während sich eine mit 70 bezeichnete nullte Ordnung weiter in dem Brillenglas 13 fortpflanzt, wodurch eine Auskopplung über einen größeren Winkelbereich erreicht werden kann. Mit 73 sind Fokuspunkte der Lichtstrahlenbündel bezeichnet, welche wiederum nahe bei einer Iris bzw. Pupille liegen.

Bei anderen Ausführungsbeispielen kann als Auskoppelelement ein optisches Element, z.B. ein in einer Folie ein belichtetes Volumenhologramm, auf das Brillenglas aufgebracht, z.B. auflaminiert werden oder auch unterhalb der Oberfläche im Inneren des Brillenglases eingeschlossen sein, um Schutz vor Umwelteinflüssen zu gewährleisten. Dabei ist das Volumenhologramm im Wesentlichen parallel zu mindestens einer Oberfläche des Brillenglases angeordnet.

Ein weiteres Ausführungsbeispiel, in welchem ebenfalls ein Gitter verwendet wird, ist in den Ausführungsbeispielen 8-10 dargestellt. Dabei ist in einem Brillenglas 13 auf einer einem Auge 10 abgewandten Seite ein Koppelelement 17, welches als Reflexionsgitter ausgestaltet ist, angeordnet. Mit 80 ist ein Bereich bezeichnet, in dem bei einer jeweiligen Stellung des Auges 10 bzw. in jeweiligen dargestellten Stellungen eine Rückwand des Auges, wo ein Retinaimplantat angeordnet sein kann, ausgeleuchtet wird.

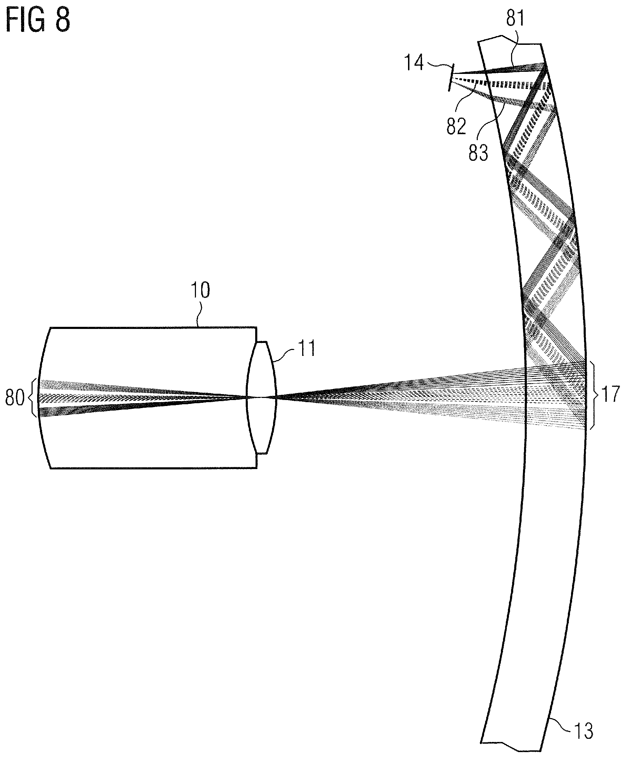

In Fig. 8 ist eine Situation dargestellt, in der das Auge 10 "geradeaus" blickt. Eine Lichtquelle 14 erzeugt infrarotes Licht, von dem als Beispiel drei Strahlenbündel 81, 82, 83 dargestellt sind, welche von verschiedenen Teilen der Beleuchtungseinrichtung 14 ausgehen und/oder verschiedene Teile eines von der IR-Lichtquelle 14 ausgehenden divergenten Strahlenbündels darstellen können.

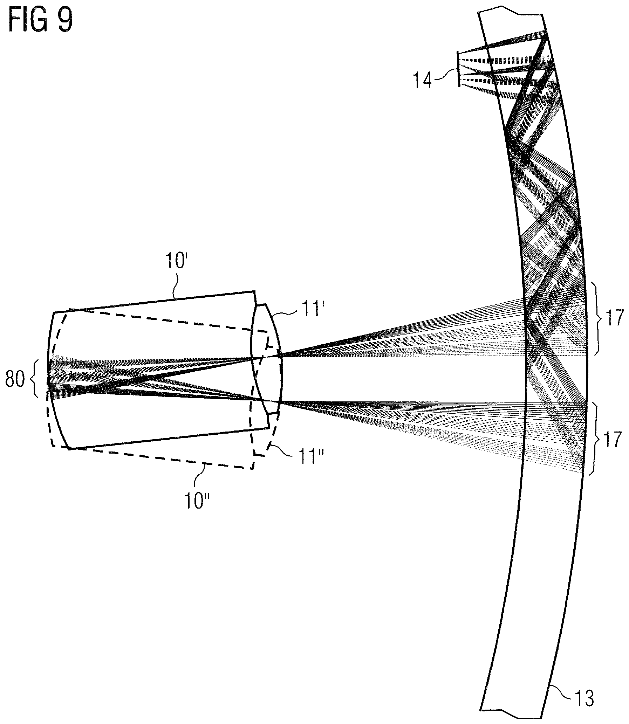

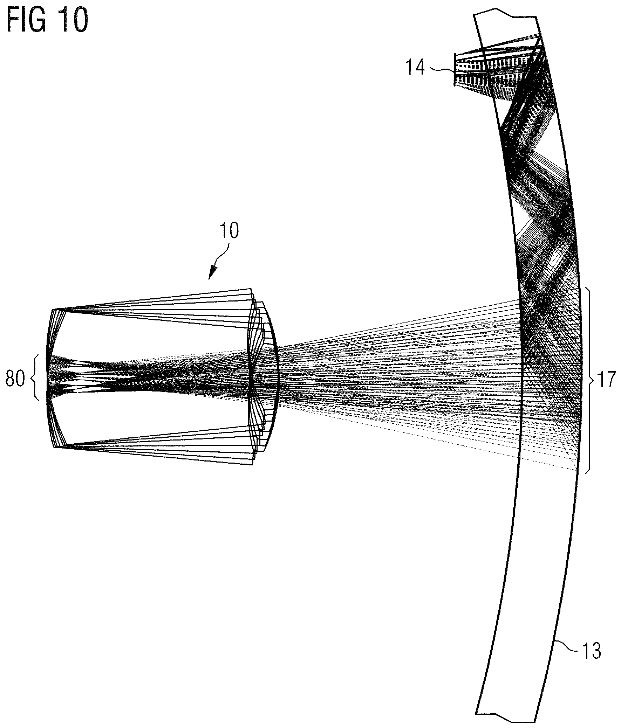

In Fig. 9 sind zwei Extrempositionen 10', 10" des Auges 10 mit entsprechenden Positionen der Pupille 11', 11" dargestellt. Zudem sind entsprechende Lichtstrahlenbündel dargestellt, welche von der Infrarotlichtquelle 14 ausgehen und von entsprechenden Bereichen der Auskoppeleinrichtung 17 in einen Bereich 80 auf der Rückwand des Auges gelenkt werden. Die Fig. 10 zeigt schließlich eine Vielzahl von Positionen des Auges 10, und einen im Wesentlichen gesamten Bereich der Auskoppeleinrichtung 17.

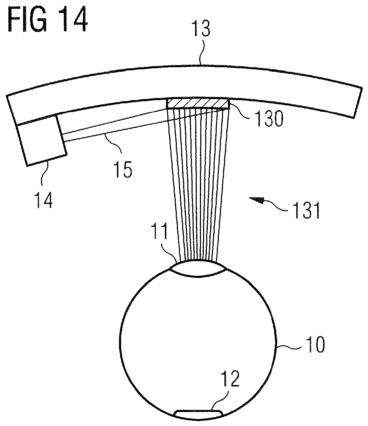

Bei den unter Bezugnahme auf die Figuren 1-9 diskutierten Ausführungsbeispielen wird Licht in ein Brillenglas eingekoppelt. Bei anderen Ausführungsbeispielen kann eine Freistrahloptik verwendet werden. Ein entsprechendes Ausführungsbeispiel ist in Fig. 14 dargestellt.

In Fig. 14 gelangt Infrarotlicht von einer Infrarotlichtquelle 14 als Freistrahl zu einer auf einer Innenseite eines Brillenglases 13 aufgebrachten oder eingearbeiteten Lenkeinrichtung 130. Die Lenkeinrichtung 130 ist bevorzugt für sichtbares Licht transparent. Die Lenkeinrichtung 130 lenkt, wie insbesondere unter Bezugnahme auf die Fig. 3-5 diskutiert, das Infrarotlicht als Vielzahl von Strahlenbündeln 131 zu dem Auge 10. Dies kann wie bereits oben diskutiert mittels eines Gitters (insbesondere Reflexionsgitter und/oder holografisches Gitter) oder eines Fresnel-Elements erfolgen. Die Erläuterungen und Details, die für eine entsprechende Auskopplung zur Berücksichtigung einer Augenbewegung oben, z.B. unter Bezugnahme auf die Fig. 3-5 gegeben wurden, sind auch auf das Ausführungsbeispiel der Fig. 14 anwendbar.

In Fig. 11 ist eine Brille 110 gemäß einem Ausführungsbeispiel dargestellt. Die Brille 110 kann ein oder zwei Brillengläser 13 wie oben unter Bezugnahme auf die Fig. 1-10 und 14 diskutiert aufweisen. Wenn nur in einem Auge ein Retinaimplantat vorhanden ist, welches zu versorgen ist, ist dabei nur ein entsprechendes Brillenglas 13 auf der entsprechenden Seite nötig. Eine Infrarotlichtquelle wie die Infrarotlichtquelle 14 kann dabei in einem Bereich 111 untergebracht sein.

Die Fig. 12 zeigt ein Flussdiagramm zur Veranschaulichung eines Verfahrens gemäß einem Ausführungsbeispiel. Das Verfahren der Fig. 12 kann insbesondere mittels der unter Bezugnahme auf die Fig. 1-11 diskutierten Vorrichtungen implementiert werden, und Details, welche unter Bezugnahme auf die Vorrichtungen besprochen wurden, können auch auf das Verfahren der Fig. 12 angewendet werden.

In einem Schritt 150 der Fig. 12 wird Infrarotlicht in ein Brillenglas eingekoppelt. Das Infrarotlicht kann beispielsweise wie beschrieben von einer Infrarotlichtquelle oder einer anderen geeigneten Lichtquelle, gegebenenfalls unter Benutzung von Filtern, erzeugt werden. In Schritt 141 wird das Licht dann zumindest teilweise koaxial zu einer Blickrichtung eines Auges, d.h. teilweise koaxial zu einer optischen Achse des Auges, ausgekoppelt. Teilweise koaxial bedeutet insbesondere, dass je nach Blickrichtung ein anderer Teil des ausgekoppelten Lichtes koaxial zur Blickrichtung liegen kann. Das Auskoppeln kann dabei wie beschrieben insbesondere derart erfolgen, dass Fokuspunkte von Strahlenbündeln von ausgekoppeltem Licht nahe einer Pupille des Auges liegen.

Wie bereits erläutert dienen die obigen Ausführungsbeispiele lediglich der Veranschaulichung und sind nicht als einschränkend auszulegen.

## Patentansprüche

1. Vorrichtung zur Versorgung eines aktiven Augenimplantats (12) in einem Auge mit Energie, umfassend:
eine Infrarotlichtquelle (14),
ein Brillenglas (13),
eine in oder auf dem Brillenglas (13) angeordnete Lenkeinrichtung (17; 130) wobei die Infrarotlichtquelle angeordnet ist, so dass von der Infrarotlichtquelle (14) ausgehendes Licht zu der Lenkeinrichtung (17; 130) gelangt, und
wobei die Lenkeinrichtung (17; 130) zum Lenken des Infrarotlichts zu einem Auge (10) eines Benutzers hin eingerichtet ist,
**dadurch gekennzeichnet, dass** die Lenkeinrichtung (17; 130) eingerichtet ist, das Infrarotlicht als ein oder mehrere fokussierte Strahlenbündel zu dem Auge (10) hin zu lenken, wobei ein oder mehrere Fokuspunkte der ein oder mehreren Strahlenbündel in einem Bereich von ±5 mm zu der Pupille des Auges (10) liegen.

2. Vorrichtung nach Anspruch 1 ,wobei die Infrarotlichtquelle (14) derart angeordnet ist, dass von der Infrarotlichtquelle (14) ausgehendes Licht in das Brillenglas (13) eingekoppelt wird, und
wobei die Lenkeinrichtung als Auskoppeleinrichtung (17) zum Auskoppeln des Infrarotlichts aus dem Brillenglas zu dem Auge (10) des Benutzers hin eingerichtet ist.

3. Vorrichtung nach Anspruch 1 oder 2, wobei die Lenkeinrichtung (17; 130) für sichtbares Licht transparent ist.

4. Vorrichtung nach einem der Ansprüche 1-3, wobei die Infrarotlichtquelle (14) im Wesentlichen nur im infraroten Bereich oberhalb von 780 nm emittiert.

5. Vorrichtung nach einem der Ansprüche 1-4, wobei die Lenkeinrichtung (17; 130) eingerichtet ist, das Infrarotlicht zumindest teilweise koaxial zu einer Blickrichtung des Auges (10) zu dem Auge (10) hin zu lenken.

6. Vorrichtung nach einem der Ansprüche 1-5, wobei die Lenkeinrichtung (17; 130) eingerichtet ist, das Infrarotlicht als eine Vielzahl von Strahlenbündeln mit unterschiedlichen Richtungen zu dem Auge (10) hin zu lenken.

7. Vorrichtung nach einem der Ansprüche 1-6, wobei die Lenkeinrichtung (17; 130) ein Transmissionsgitter, ein Reflexionsgitter, einen wellenlängenselektiven Strahlteiler, ein Volumenhologramm und/oder ein Fresnel-Element umfasst.

8. Vorrichtung nach Anspruch 7, wobei das Transmissionsgitter und/oder das Reflexionsgitter ein holografisches Gitter umfasst.

9. Verfahren zur Versorgung eines aktiven Augenimplantats (12) in einem Auge mit Energie,
umfassend:
Lenken von Infrarotlicht zu einer Lenkeinrichtung (17;130) in oder auf einem Brillenglas, und
Lenken des Infrarotlichts von der Lenkeinrichtung (17;130) zu einem Auge (10) eines Benutzers hin,
**dadurch gekennzeichnet, dass** das Infrarotlicht von der Lenkeinrichtung (17; 130) als ein oder mehrere fokussierende Strahlenbündel zu dem Auge (10) hingelenkt wird, wobei ein oder mehrere Fokuspunkte der ein oder mehreren Strahlenbündel in einem Bereich von ±5 mm zu der Pupille des Auges (10) liegen.

10. Verfahren nach Anspruch 9,
wobei das Lenken von Infrarotlicht zu der Lenkeinrichtung ein Einkoppeln des Infrarotlichts in ein Brillenglas umfasst, und
wobei das Lenken des Infrarotlichts von der Lenkeinrichtung ein Auskoppeln des Infrarotlichts zu einem Auge eines Benutzers hin umfasst.

11. Verfahren nach Anspruch 9 oder 10, wobei das Lenken des Infrarotlichts von der Lenkeinrichtung ein Lenken von verschiedenen Stellen des Brillenglases unter verschiedenen Winkeln zu dem Auge (10) hin umfasst.

## Claims

1. Apparatus for supplying power to an active ocular implant (12) in an eye, comprising:
an infrared light source (14),
a spectacle lens (13),
a steering device (17; 130) arranged in or on the spectacle lens (13),
wherein the infrared light source is arranged such that light emanating from the infrared light source (14) reaches the steering device (17; 130), and
wherein the steering device (17; 130) is configured to steer the infrared light towards an eye (10) of a user,
**characterized in that** the steering device (17; 130) is configured to steer the infrared light towards the eye (10) in the form of one or more focused beams, wherein one or more focal points of the one or more beams are in a range of ±5 mm from the pupil of the eye (10).

2. Apparatus according to Claim 1, wherein the infrared light source (14) is arranged in such a way that light emanating from the infrared light source (14) is input coupled into the spectacle lens (13), and
wherein the steering device is configured as an output coupling device (17) for output coupling the infrared light from the spectacle lens towards the eye (10) of the user.

3. Apparatus according to Claim 1 or 2, wherein the steering device (17; 130) is transparent to visible light.

4. Apparatus according to any of Claims 1-3, wherein the infrared light source (14) substantially only emits in the infrared range above 780 nm.

5. Apparatus according to any of Claims 1-4, wherein the steering device (17; 130) is configured to steer the infrared light towards the eye (10) in a manner at least partly coaxial to a viewing direction of the eye (10).

6. Apparatus according to any of Claims 1-5, wherein the steering device (17; 130) is configured to steer the infrared light towards the eye (10) as a multiplicity of beams with different directions.

7. Apparatus according to any of Claims 1-6, wherein the steering device (17; 130) comprises a transmission grating, a reflection grating, a wavelength-selective beam splitter, a volume hologram, and/or a Fresnel element.

8. Apparatus according to Claim 7, wherein the transmission grating and/or the reflection grating comprises a holographic grating.

9. Method for supplying power to an active ocular implant (12) in an eye, comprising:
steering infrared light to a steering device (17; 130) in or on a spectacle lens, and
steering the infrared light from the steering device (17; 130) towards an eye (10) of a user,
**characterized in that** the infrared light is steered towards the eye (10) in the form of one or more focused beams by the steering device (17; 130), wherein one or more focal points of the one or more beams are in a range of ±5 mm from the pupil of the eye (10).

10. Method according to Claim 9,
wherein steering infrared light to the steering device comprises an input coupling of the infrared light into a spectacle lens, and
wherein steering the infrared light from the steering device comprises an output coupling of the infrared light towards an eye of a user.

11. Method according to Claim 9 or 10, wherein steering the infrared light from the steering device comprises steering towards the eye (10) at different angles from different positions of the spectacle lens.

## Revendications

1. Dispositif d'alimentation en énergie d'un implant oculaire actif (12) dans un œil, comprenant :
une source de lumière infrarouge (14),
un verre de lunettes (13),
un module de direction (17 ; 130) disposé dans ou
sur le verre de lunettes (13)
la source de lumière infrarouge étant disposée de telle sorte que la lumière sortant de la source de lumière infrarouge (14) atteigne le module de direction (17 ; 130), et
le module de direction (17 ; 130) étant conçu pour diriger la lumière infrarouge vers un œil (10) d'un utilisateur,
**caractérisé en ce que** le module de direction (17 ; 130) est conçu pour diriger la lumière infrarouge sous la forme d'un ou plusieurs faisceaux de rayons focalisés vers l'œil (10), un ou plusieurs points focaux des un ou plusieurs faisceaux de rayons étant situés dans une zone de ± 5 mm par rapport à la pupille de l'œil (10).

2. Dispositif selon la revendication 1, la source de lumière infrarouge (14) étant disposée de manière à ce que la lumière sortant de la source de lumière infrarouge (14) soit injectée par couplage dans le verre de lunettes (13), et
le module de direction étant conçu comme un module de sortie par couplage (17) destiné à sortir par couplage la lumière infrarouge du verre de lunettes pour l'amener à l'œil (10) de l'utilisateur.

3. Dispositif selon la revendication 1 ou 2, le module de direction (17 ; 130) étant transparent pour la lumière visible.

4. Dispositif selon l'une des revendications 1 à 3, la source de lumière infrarouge (14) n'émettant sensiblement que dans le domaine infrarouge supérieur à 780 nm.

5. Dispositif selon l'une des revendications 1 à 4, le dispositif de direction (17 ; 130) étant conçu pour diriger la lumière infrarouge vers l'œil (10) au moins en partie coaxialement à une direction d'observation de l'œil (10).

6. Dispositif selon l'une des revendications 1 à 5, le module de direction (17 ; 130) étant conçu pour diriger la lumière infrarouge vers l'œil (10) sous la forme d'un grand nombre de faisceaux de rayons ayant des directions différentes.

7. Dispositif selon l'une des revendications 1 à 6, le module de direction (17 ; 130) comprenant un réseau de transmission, un réseau de réflexion, un séparateur de faisceau sélectif en longueur d'onde, un hologramme de volume et/ou un élément de Fresnel.

8. Dispositif selon la revendication 7, le réseau de transmission et/ou le réseau de réflexion comprenant un réseau holographique.

9. Procédé d'alimentation en énergie d'un implant oculaire actif (12) dans un œil, ledit procédé comprenant les étapes suivantes :
diriger la lumière infrarouge vers un module de direction (17 ; 130) dans ou sur un verre de lunettes, et
diriger la lumière infrarouge du module de direction (17 ; 130) vers un œil (10) d'un utilisateur, **caractérisé en ce que** la lumière infrarouge est dirigée du module de direction (17 ; 130) vers l'œil (10) sous la forme d'un ou plusieurs faisceaux de rayons focalisés, un ou plusieurs points focaux des un ou plusieurs faisceaux de rayons étant situés dans une zone de ± 5 mm par rapport à la pupille de l'œil (10).

10. Procédé selon la revendication 9,
le fait de diriger la lumière infrarouge vers le module de direction incluant l'injection par couplage de la lumière infrarouge dans un verre de lunettes, et
le fait de diriger la lumière infrarouge depuis le module de direction incluant une sortie par couplage de la lumière infrarouge vers un œil d'un utilisateur.

11. Procédé selon la revendication 9 ou 10, le fait de diriger la lumière infrarouge depuis le module de direction incluant une direction depuis différents emplacements du verre de lunettes sous différents angles vers l'œil (10).
